# EUROPEAN PATENT SPECIFICATION

(11) **EP 1 571 929 B1**
(45) Date of publication and mention of the grant of the patent: **23.07.2008**
(21) Application number: 02781668.5
(22) Date of filing: 18.12.2002
(51) Int. Cl.: A23L 3/3463, A23L 3/3472

(54) **A PROCESS FOR THE PREPARATION OF ANTIOXIDANT CONSERVE FROM INDIAN CURRY LEAVES (MURRAYA KOENIGII SPRENG.)**
VERFAHREN ZUR HERSTELLUNG VON ANTIOXIDANTEN AUS CURRY-BLÄTTERN (MURRAYA KOENIGII SPRENG.)
PROCEDE DE PREPARATION D'UN CONSERVATEUR ANTIOXYDANT A PARTIR DE FEUILLES DE CURRY (MURRAYA KOENIGII SPRENG.)

(43) Date of publication of application: 14.09.2005
(73) Proprietor: COUNCIL OF SCIENTIFIC AND INDUSTRIAL RESEARCH, New Delhi 100 001 (IN)
(72) Inventor: RAO, Lingamallu Jagan Mohan, 570 013 Karnataka (IN); RAMALAKSHMI, Kulathooran, 570 013 Karnataka (IN); BORSE, Babasaheb Bhaskarrao, 570 013 Karnataka (IN); RAGHAVAN, Bashyam, 570 013 Karnataka (IN)
(74) Representative: Mattsson, Niklas
(86) International application number: PCT/IB2002/005445
(87) International publication number: WO 2004/054391

(56) References cited:
- WO-A-02/32440
- US-A- 4 380 506
- TACHIBANA Y.: "Antioxidative activity of carbazoles from Murraya koenigii leaves" JOURNAL OF AGRICULTURAL AND FOOD CHEMISTRY, vol. 49, no. 11, 2001, pages 5589-5594, XP002250357
- RAMSEWAK R.: "Biologically active carbazole alkaloids from Murraya koenigii" JOURNAL OF AGRICULTURAL AND FOOD CHEMISTRY, vol. 47, no. 2, 1999, pages 444-447, XP002250358
- PATENT ABSTRACTS OF JAPAN vol. 1997, no. 01, 31 January 1997 (1997-01-31) -& JP 08 225420 A (INAHATA KORYO KK;NIPPON FUNMATSU YAKUHIN KK), 3 September 1996 (1996-09-03)
- PATENT ABSTRACTS OF JAPAN vol. 1997, no. 01, 31 January 1997 (1997-01-31) -& JP 08 242814 A (INAHATA KORYO KK), 24 September 1996 (1996-09-24)
- SETHI V: "EFFECT OF ADDITION OF SPICE EXTRACTS AND FLAVOURING COMPOUNDS ON THE KEEPING QUALITY OF TOMATO JUICE STORED IN DIFFERENT CONTAINERS" INDIAN FOOD PACKER, BOMBAY, IN, vol. 45, no. 3, 1991, pages 17-18, XP000946721 ISSN: 0019-4808 cited in the application
- KHAN B A ET AL: "ANTI-OXIDANT EFFECTS OF CURRY LEAF, MURRAYA KOENIGII AND MUSTARD SEEDS, BRASSICA JUNCEA IN RATS FED WITH HIGH FAT DIET" INDIAN JOURNAL OF EXPERIMENTAL BIOLOGY, XX, XX, vol. 35, no. 2, February 1997 (1997-02), pages 148-150, XP001000380 ISSN: 0019-5189

## Description

### Technical Field

The present invention relates to a simple process for the preparation of safe, environmental friendly, and natural antioxidant conserve of activity ranging between 80-85%, and use of antioxidant conserve at concentration ranging between ranging between 0.2 to 3.0%, comprising oleoresin from Indian curry leaves (*Murraya Koenigii Spreng.)* as an anti-oxidant in food and pharmaceutical substances and additives.

### Background Art

Antioxidants play a crucial role in preventing or delaying autoxidation and have attracted a lot of attention as food additives. The deterioration of food with time, results largely from its biological nature and is inevitable. During the production, processing, distribution and storage prior to actual consumption, food undergoes various modes of deterioration that involve biological changes by microbes as well as chemical changes. The latter is ascribed to enzymatic and non-enzymatic oxidation of lipids and phenolics, which cause undesirable changes in flavour, appearance, physical character, nutritional value and toxicity. Deoxygenation, airtight packing, and other techniques have solved some of these problems to a certain extent, but the role of antioxidants as either food constitutents or as additives cannot be overlooked. Both synthetic and natural antioxidants are widely used in many food products.

The subject of natural antioxidants has been developing since past decade, mainly because of the increasing limitations on the use of synthetic antioxidants and enhanced public awareness of health issues. In general, consumer prefers natural antioxidants because these are considered safe and environmental friendly. Commercial antioxidants are generally synthetic compounds. There has been a growing interest in replacing them with natural ingredients due to possible toxicity of synthetic antioxidants (Chang et al J.Food Sci. 42, 1102, 1977) as shown by clinical studies (Toxicological aspects of antioxidants used as food additives, In Food antioxidants; Hudson,B.J.F., Ed.; Elsevier; London, 1990; p253). Further, the use of some common synthetic antioxidants such as butylated hydroxy anisole (BHA) and butylated hydroxy toluene (BHT) has become controversial issue because of adverse toxicological data (Food antioxidants, edited by Madhavi, Despande and Salunke, Marcel Dekker, Inc. Newyork 1995, p.267) and the use of BHT is already banned in India. The use of natural antioxidants in food is limited due to the lack of knowledge about their molecular compositions, the content of active compounds in the raw materials and the availability of relevant toxicological data. Hence, evaluation of the antioxidative activity of naturally occurring substances has been of interest in recent years *(*Nahrung, 40, 261, 1996). Currently, the use of some naturally occurring antioxidants in preventive and therapeutic medicine is gaining importance.

Literature survey revealed that there is no process for the isolation antioxidant conserve from Indian Curry leaves ***(Murraya koenigii* Spreng.)**

WO 0232440 discloses the use and preparation of a natural antioxidant composition for food or pharmaceutical compositions. In a process for preparing an extract, Murray koenigii leaves are washed, dried, powdered, extracted with polar solvent and the solvent is then evaporated.

Curry leaf (*Murraya koenigii* Spreng) is an aromatic small tree belonging to citrus family Rutaceae and occurs widely in East Asia. Its leaves have slightly pungent, bitter and feebly acidulous taste and these characterstics are retained after drying. The chemical composition of volatile oil and the changes during dehydration were studied by Prakash and Natarajan (J.Fd.Sci.Technol., 11, 284-286, 1974). Curry leaf, at 1% level gave better protection to ghee than when BHA or BHT at 0.02% as specified by PFA. Ghee with peroxide value of 0.4 gave after storage of 5 months, 1.56 with 0.02% BHA or BHT and 1.50 with 0.5% curry leaf. (Patel and Rajorhia, J.Fd.Sci.Technol., 16, 158, 1979). The shelf life of tomato juice was extended by incorporating aqueous extract of curry leaf at 0.5%. (Vijay Sethi, Indian Food Packer, 45, 17-18, 1991).

### Objects of the present invention

The main object of the present invention is to develop a simple process for the preparation of safe, environmental friendly, and natural antioxidant conserve comprising oleoresin from Indian curry leaves *(Murraya Koenigii Spreng.)*

Another main object of the present invention is to develop a process for obtaining antioxidant conserve comprising oleoresin.

Yet another object of the present invention to develop a process for obtaining high yield antioxidant of range between 7-10% of the weight of the leaves.

Still another object of the present invention is to obtain the anti-oxidant activity of the extract is ranging between 80 to 85% from Indian curry leaves.

Another main object of the present invention is to develop a use of antioxidant conserve comprising oleoresin from Indian curry leaves *(Murraya Koenigii Spreng.)* as an anti-oxidant in food and pharmaceutical substances and additives.

### Summary of the present invention

The present invention relates to a simple process for the preparation of safe, environmental friendly, and natural antioxidant conserve of activity ranging between 80-85%, comprising oleoresin from Indian curry leaves *(Murraya Koenigii Spreng.),* said process-comprising steps of drying the washed curry leaves at temperature ranging between 30-80°C, for time duration ranging between 2-10 hours, powdering the dried leaves into coarse powder form, extracting the powder with polar solvent preferably ketone with alkyl group, wherein the ratio of leaves to solvent is in the range of 1:5-1:7 and obtaining antioxidant conserve comprising oleoresin from the extract by removing the solvent; also, use of antioxidant conserve at concentration ranging between ranging between 0.2 to 3.0%, comprising oleoresin from Indian curry leaves (*Murraya Koenigii Spreng*.) as an anti-oxidant in food and pharmaceutical substances and additives.

### Detailed description of the present invention

Accordingly, the present invention relates to a simple process for the preparation of safe, environmental friendly, and natural antioxidant conserve of activity ranging between 80-85%, comprising oleoresin from Indian curry leaves *(Murraya Koenigii Spreng.),* said process-comprising steps of drying the washed curry leaves at temperature ranging between 30-80°C, for time duration ranging between 2-10 hours, powdering the dried leaves into coarse powder form, extracting the powder with polar solvent preferably ketone with alkyl group, wherein the ratio of leaves to solvent is in the range of 1:5-1:7 and obtaining antioxidant conserve comprising oleoresin from the extract by removing the solvent; also described is use of antioxidant conserve at concentration ranging between ranging between 0.2 to 3.0%, comprising oleoresin from Indian curry leaves *(Murraya Koenigii Spreng.)* as an anti-oxidant in food and pharmaceutical substances and additives.

A simple process for the preparation of safe, environmental friendly, and natural antioxidant conserve comprising oleoresin from Indian curry leaves (*Murraya Koenigii Spreng.),* said process comprising steps of:
- drying the washed curry leaves at temperature ranging between 30-80°C, for time duration ranging between 2-10 hours.
- powdering the dried leaves into coarse powder form,
- extracting the powder with polar solvent preferably ketone with alkyl group, wherein the ratio of leaves to solvent is in the range of 1:5-1:7,
- obtaining antioxidant conserve comprising oleoresin from the extract by removing the solvent.

In an embodiment of the present invention, wherein the solvent is acetone and ethylmethyl ketone.

In another embodiment of the present invention, wherein removal of the solvent is at the temperature ranging between 10-40°C.

In yet another embodiment of the present invention, wherein yield of the antioxidant is ranging between 7-10% of the weight of the leaves.

In the present invention, the ratio of leaves to solvent is ranging between 1:5 to 1:7.

In still another embodiment of the present invention, wherein the anti-oxidant activity of the extract is ranging between 80 to 85%.

In another main embodiment of the present invention, wherein use of antioxidant conserve comprising oleoresin from Indian curry leaves *(Murraya Koenigii Spreng.)* as an anti-oxidant in food and pharmaceutical substances and additives.

In still another embodiment of the present invention, wherein the anti-oxidant activity of the extract is ranging between 80 to 85%.

In still another embodiment of the present invention, wherein the concentration of the extract in food and pharmaceutical is ranging between 0.2 to 3.0%.

In still another embodiment of the present invention, wherein the said use shows no toxicity.

In still another embodiment of the present invention, wherein the said use does not affect the taste of the food and pharmaceutical substances.

In still another embodiment of the present invention, wherein the said use is environmental friendly.

In still another embodiment of the present invention, the yield of the antioxidant conserve in step (v) is 8.5% by chromatography method with respect to the weight of dehydrated curry leaves.

In still another embodiment of the present invention the antioxidant activity of the conserve is comparable to the standard compound BHA.

The process for the preparation of antioxidant conserves from the Indian Curry leaves was carried out according to the following flow diagram.

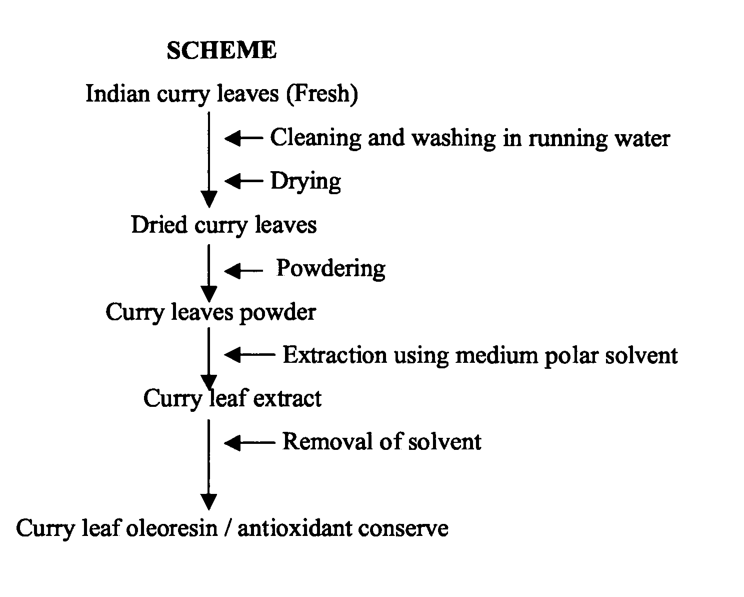

### Antioxidant assay by β-carotene-linoleate model system

The antioxidant activity of curry leaves extract was evaluated by the β-carotene-linoleate model (Jayaprakasha and Jaganmohan Rao, Z.Naturforsch. 55C, 1018-1022, 2000). 0.2 mg of the β-carotene in 0.5 ml of chloroform, 20 mg of linoleic acid and 200 mg of Tween-40 (polyoxyethylene sorbitan monopalmitate) were mixed together. The chloroform was removed at 40 °C under vacuum using a rotary evaporator. The resulting solution was immediately diluted with 10 ml of triple-distilled water and the emulsion was mixed well for 1 min. The emulsion was further diluted with 40 ml of oxygenated water before use. 4 ml aliquots of this mixture were transferred into different tubes containing 0.2 ml of samples at 100 ppm concentrations in ethanol, butylated hydroxyanisole (BHA) was used for comparative purposes. A control containing 0.2 ml of ethanol and 4 ml of the above mixture was prepared. Optical density (OD) at 470 nm were taken for the all extracts and pure compounds immediately (*t* = 0) at 15 min intervals for 1.5 h (*t* = 90). The tubes were incubated at 50 °C in a water bath. All determinations were performed in triplicate. Measurement of OD was continued until the colour of β-carotene disappeared in the control. The antioxidant activities (AA) of the samples were evaluated in terms of bleaching the β-carotene using the following formula. AA = 100[1-(Aₒ-Aₜ)/(A^{o}ₒ - A^{o}ₜ)] where Aₒ and A^{o}ₒ are the OD measured at zero time of incubation for test sample and control, respectively. Aₜ and A^{o}ₜ are the OD measured in the test sample and control, respectively, after incubation for 90 min.

### The advantages of the process are:

This is the first report of a process for the isolation of antioxidant conserves from the Indian curry leaves.

The acetone extract of curry leaves could be used as natural antioxidant conserve in food systems. Indian curry leaves are a source of natural antioxidant. This process will help in the proper utilisation of Indian curry leaves. This process will add to the economics of cultivator of Indian curry leaves.

### The novelty of the process:

1. Isolation of antioxidant conserve from Indian curry leaves.
2. Separation of antioxidant conserves using simple extraction techniques.

The following examples are given by way of illustrations of the present invention and therefore should not be construed to limit the scope of the present invention.

### Example-1

Indian curry leaves were cleaned and washed with running water. These were dried in a cross flow dryer at 55 ± 5 °C for 6±1hr. Dried leaves are powdered to obtain coarse powder. Dry powder (500g) extracted with ethylmethylketone / acetone in a glass column. The ratio of leaves to eluting solvent is 1: 6±1. Solvent is removed from the eluate at 35±5°C. The yield of the conserve 1 is 7.25%.

### Example-2

Indian curry leaves were cleaned and washed with running water. These were dried in a cross flow dryer at 55 ± 5 °C for 6±1hr. Dried leaves are powdered to obtain coarse powder. Dry powder (250g) extracted with ethylmethylketone / acetone in a glass column. The ratio of leaves to eluting solvent is 1: 6±1. Solvent is removed from the elute at 35±5°C. The yield of the conserve 2 is 8.5%.

The antioxidant activities of conserves 1 and 2 are 83.1% and 83.2% at 100 ppm concentration and comparable to the antioxidant activity of BHA (90.2%) at the same concentration.

### Example 3

The said conserve of Indian curry leaves was tried as an anti-oxidant in food and pharmaceutical substances and additives. Some of the food systems used tomato, apples, potato, pickles, fruit jams, soft drinks, ketchups, etc. Further, the pharmaceutical product comprises analgesics, anti-pyretic, etc.

Here, concentration of the conserve in food and pharmaceutical is ranging between 0.2 to 3.0%. Further, the anti-oxidant activity of the extract is found to be ranging between 80 to 85%.

In addition, the food and pharmaceutical items with said conserve as anti-oxidant with food preservation activity was found to be non-toxic. Also, the taste of the food and pharmaceutical products was not affected by adding this extract

## Claims

1. A simple process for the preparation of safe, environmental friendly, and natural antioxidant conserve comprising oleoresin from Indian curry leaves *(Murraya Koenigii Spreng.),* said process comprising steps of:
a. drying the washed curry leaves at temperature in the range of 30-80°C, for time duration ranging between 2-10 hours.
b. powdering the dried leaves into coarse powder form,
c. extracting the powder with polar solvent preferably ketone with alkyl group, wherein the ratio of leaves to solvent is in the range of 1:5-1:7.
d. obtaining antioxidant conserve comprising oleoresin from the extract by removing the solvent.

2. A process as claimed in claim 1, wherein the solvent is acetone and ethylmethyl ketone.

3. A process as claimed in claim 1, wherein removal of the solvent is at the temperature in the range of 10-40°C.

4. A process as claimed in claim 1, wherein the anti-oxidant activity of the extract is in the range of 80 to 85%.

## Patentansprüche

1. Einfaches Verfahren zur Herstellung von sicherem, umweltfreundlichem und natürlichem Antioxidationsmittel-Präparat (antioxidant conserve), umfassend Fettharz aus indischen Curryblättern (Murraya Koenigii Spreng.), wobei das Verfahren die Schritte umfasst:
a) Trocknen der gewaschenen Curryblätter bei einer Temperatur im Bereich von 30 bis 80°C für eine Zeitdauer von 2 bis 10 Stunden.
b) Pulverisieren der getrockneten Blätter in eine grobe Pulverform,
c) Extrahieren des Pulvers mit polaren Lösungsmittel vorzugsweise ein Keton mit Alkylgruppe, wobei das Verhältnis von Blättern zu Lösungsmittel im Bereich von 1 zu 5 bis 1 zu 7 ist.
d) Erhalten des Antioxidationsmittel-Präparats (antioxidant conserve) umfassend Fettharz aus dem Extrakt durch Entfernen des Lösungsmittels.

2. Verfahren nach Anspruch 1, wobei das Lösungsmittel Aceton und Ethylmethylketon ist.

3. Verfahren nach Anspruch 1, wobei das Lösungsmittel bei einer Temperatur im Bereich von 10 bis 40°C entfernt wird.

4. Verfahren nach Anspruch 1, wobei die Antioxidationsaktivität des Extraktes im Bereich von 80 bis 85% liegt.

## Revendications

1. Procédé simple pour la préparation d'une conserve anti-oxydante inoffensive, favorable à l'environnement et naturelle comprenant une oléorésine provenant de feuilles de curry indien (Murraya Koenigii Spreng.), ledit procédé comprenant les étapes de :
a. séchage des feuilles de curry lavées à une température dans la plage de 30-80°C pendant une durée entre 2-10 h,
b. réduction en poudre des feuilles séchées sous forme d'une poudre grossière,
c. extraction de la poudre avec un solvant polaire, de préférence une cétone ayant un groupe alkyle où le rapport des feuilles au solvant est dans la plage de 1:5-1:7,
d. obtention d'une conserve anti-oxydante comprenant une oléorésine provenant de l'extrait par retrait du solvant.

2. Procédé selon la revendication 1, où le solvant est l'acétone et la méthyléthylcétone.

3. Procédé selon la revendication 1, où le retrait du solvant est à une température dans la plage de 10-40°C.

4. Procédé selon la revendication 1, où l'activité anti-oxydante de l'extrait est dans la plage de 80 à 85 %.
